(19) Europäisches Patentamt
European Patent Office
Office européen des brevets

(11) **EP 2 775 983 B1**

(12) **EUROPEAN PATENT SPECIFICATION**

(45) Date of publication and mention
of the grant of the patent:
**09.03.2016 Bulletin 2016/10**

(21) Application number: **11785260.8**

(22) Date of filing: **10.11.2011**

(51) Int Cl.:
***A61G 11/00*** (2006.01)

(86) International application number:
**PCT/US2011/060190**

(87) International publication number:
**WO 2013/070229 (16.05.2013 Gazette 2013/20)**

(54) **WARMING THERAPY DEVICE INCLUDING MATTRESS TEMPERATURE CORRECTION**

WÄRMETHERAPIEVORRICHTUNG MIT MATRATZENTEMPERATURKORREKTUR

DISPOSITIF DE THÉRAPIE DE RÉCHAUFFEMENT COMPRENANT UNE CORRECTION DE TEMPÉRATURE DE MATELAS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**17.09.2014 Bulletin 2014/38**

(73) Proprietor: **Draeger Medical Systems, Inc.**
**Telford, PA 18969 (US)**

(72) Inventors:
• **TIMME, Ulf**
**Havertown, PA 19083 (US)**
• **GERSHTEYN, Jacob**
**Newtown, PA 18940 (US)**

(74) Representative: **McCartney, Jonathan William**
**Haseltine Lake LLP**
**Redcliff Quay**
**120 Redcliff Street**
**Bristol BS1 6HU (GB)**

(56) References cited:
**US-A- 3 920 000     US-A- 5 730 355**

**Description**

**FIELD OF THE INVENTION**

This present invention relates generally to a method and apparatus for performing warming therapy on medical patients. Most particularly, the present invention relates to a method and apparatus for adjusting the temperature within an enclosure while accounting for the humidity level.

**BACKGROUND OF THE INVENTION**

It is known to control both the temperature and relative humidity of the air adjacent a medical patient (e.g., infant) in a warming therapy device. For example, many warming therapy devices (e.g., incubators, warmers, etc.) include systems which are designed to control the temperature and humidity of the environment surrounding an infant patient disposed inside a hood of the warming therapy device. To control the temperature and humidity within the warming therapy device, the existing temperature and humidity are sensed and then adjusted. The temperature within the warming therapy device may be adjusted using a heating element located within the air circulation system of the warming therapy device, which is responsive to a temperature sensor. The humidity within a warming therapy device may be adjusted using a humidification device that is adapted to inject humidity into the air circulation system of the warming therapy device.
Humidification systems typically comprise at least a water reservoir, a boiling chamber, and a heating element. The heating element typically acts to heat the water within the boiling chamber, and thus create humidity by adding water vapor to heated air. The temperature and humidity concentration of the air within a warming therapy device are typically controlled by a forced air circulation system, where the impeller of a fan unit recirculates the air. Temperature is generally measured with a sensor located inside of the incubator compartment. However, the temperature measurement can be incorrect due to the changing density of the air resulting from higher humidity levels inside of the incubator. Because one of the objectives of a warming therapy device is to create an environment with a specific prescribed temperature level, which requires accurate measurements of the temperature inside the enclosure, there is a need to correct temperatures which may not be accurately measured due to environmental conditions.

SUMMARY OF THE INVENTION

The invention is defined in the attached independent claims, to which reference should now be made. Further, optional features are defined in the subclaims appended thereto.
In first exemplary embodiment, the present invention comprises a warming therapy apparatus, including a base for supporting a medical patient, a hood surrounding a portion of the base, a heater for heating the air inside the hood, a sensor for measuring an air temperature and a humidity inside the hood, and a controller for adjusting the air temperature based on the measured humidity and a correction factor.
In a another exemplary embodiment, the present invention comprises a method of adjusting the air temperature inside an enclosure for medical patients, including the steps of measuring the air temperature at a predetermined location inside the enclosure, measuring the humidity inside the enclosure, determining a correction factor, applying the correction factor to the measured air temperature to determine a temperature correction value, and adjusting the air temperature inside the enclosure based on the temperature correction value.

BRIEF DESCRIPTION OF THE DRAWINGS

Figure 1 is an exploded perspective view of a warming therapy device according to an exemplary embodiment of the present invention.
Figure 2 is a system for adjusting the air temperature by accounting for changes in air density caused by humidity.
Figure 3 is a process for adjusting the temperature of the air within an enclosure of a warming therapy device by accounting for changes in air density caused by humidity.

DETAILED DESCRIPTION

The present invention relates to a warming therapy device (e.g. incubator, warmer, etc.) including a humidification system and a temperature adjustment system which accurately measures the temperature of the air inside the warming therapy device.
By way of example, the temperature adjustment system according to an embodiment of the present invention may be used in connection with an incubator and temperature sensor such as are disclosed and discussed in U.S. Pat No. 5,730,355 (assigned to the same assignee as the present application). For example, U.S. Pat, No. 5,730,355 describes an incubator having a base 16 and a sensor module 44 for sensing conditions such as air temperature and humidity within the incubator, and providing control signals for regulating the same.

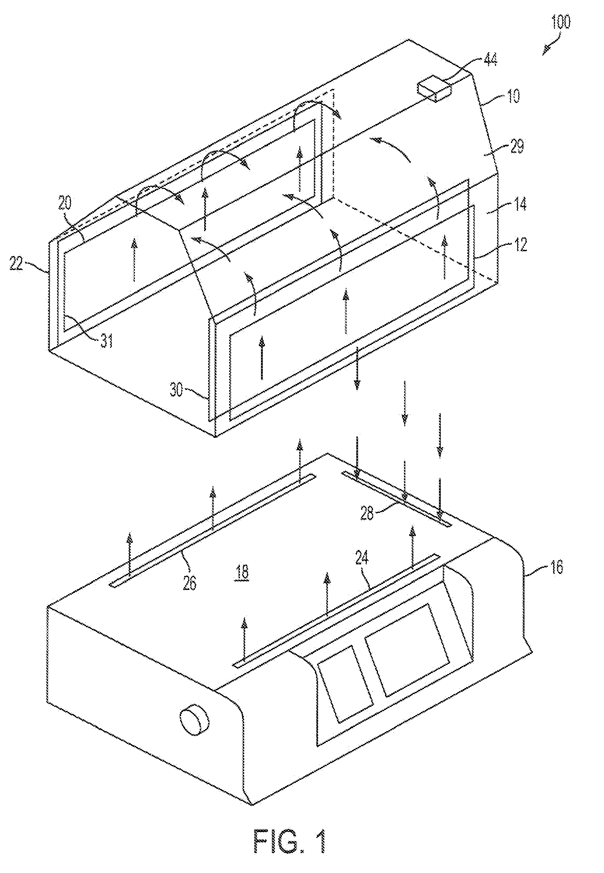

Closed care warming therapy devices (e.g., incubators) provide physical separation between the environment where the infant patient is disposed and the surrounding ambient air. This separation is typically provided by a hood or similar member which encloses the infant patient therein. This encapsulation of the infant patient facilitates creation of conditions favorable for the infant patient's development. Often times, the conditions inside the hood can be significantly different from those present in the ambient environment. Conditions inside the hood may be determined by varying the temperature level and/or humidity concentration within the closed care environment, all of which can be controlled automatically using sensors integrated in the warming therapy device. For example, the temperature within the closed care environment may be controlled by sensing the skin temperature of the infant patient or the temperature at a location within the hood and making appropriate adjustments. Figure 1, discussed below, show an exemplary closed care warming therapy device.

Medical patients are generally placed on a mattress in the base of a warming therapy device. In addition, sensors are generally placed on the hood of a warming therapy device to measure the temperature of the air inside the enclosure. Due to the differences in location between the sensor and mattress, temperature measurements of the air inside the warming therapy device may differ. The sensor measures the air within a warming therapy device. A controller applies an offset to account for the difference in air temperatures between the location of the sensor and the medical patient. However, the sensor does not account for incorrect air temperature measurements due to changing air density resulting from humidity fluctuations in the warming therapy device. Accordingly, the present invention provides systems and methods for correcting the air temperature such that it accurately mirrors the air temperature as measured in the vicinity of a mattress in a warming therapy device by adjusting the air temperature based on humidity in the warming therapy device.

Figure 1 provides a closed care warming therapy device 100 comprising a hood 10 and a base 16. The hood 10 has access door 12 in wall 14 and access door 20 in wall 22, for example. The hood 10 is mounted on base 16, which comprises a deck 18. The area between the inside of hood 10 and deck 18, when the hood 10 is placed on the base 16, defines the enclosure. The deck 18 includes openings 24, 26, 28 which allow for air to enter and leave the enclosure as indicated by the arrows leaving openings 24, 26 and entering opening 28.

When the door 12 of the hood 10 is opened, the air passing through opening 24 in deck 18 forms a warm air curtain, which serves as a barrier and reduces the effect of the ambient temperature outside the incubator on the enclosure area. When door 20 of hood 10 is opened, the air passing through opening 26 in deck 18 forms a warm air curtain, similar to when door 12 is opened.

The warming therapy device 100 further comprises a first inner wall 30 and a second inner wall 31, secured to doors 12 and 20, respectively- The first inner wall 30 and the second inner wall 31 are moveable with their corresponding doors 12, 20. The inner walls 30, 31 reduce radiant heat losses when the respective access doors are closed and serve to carry warm air introduced through openings 24, 26 to the top of hood 10 as shown by the arrows inside of the hood 10.

The hood 10 also includes a sensor 44 which is able to measure the temperature of the air and humidity inside the enclosure.

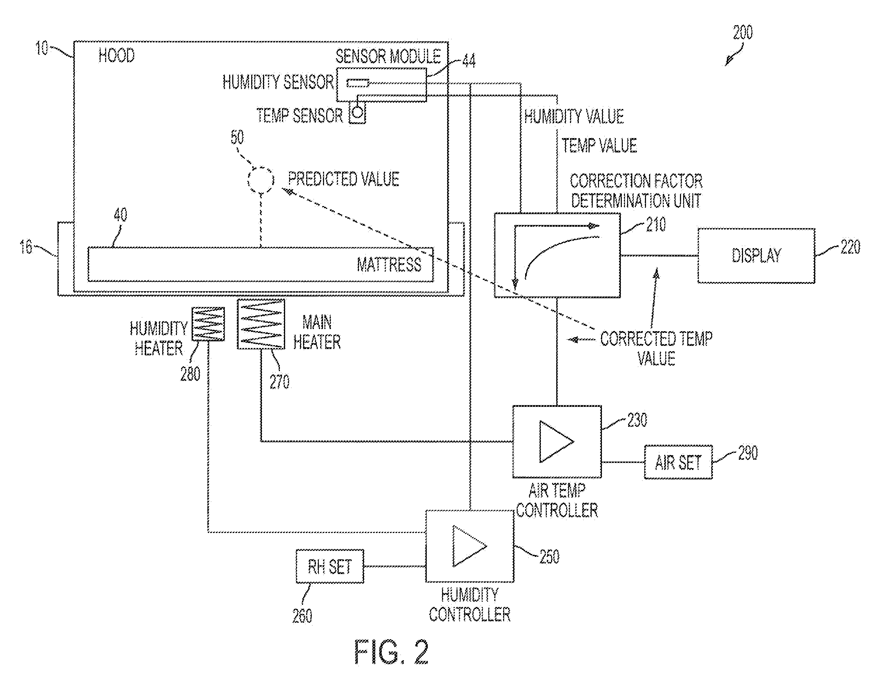

Figure 2 illustrates the system 200 for adjusting the temperature of the air inside the enclosure by accounting for effects on the measured temperature of the air inside the enclosure between the hood 10 and base 20 caused by changes in the density of air due to fluctuations in humidity within the enclosure. System 200 includes a warming therapy device 100 comprising a hood 10, which includes a sensor 44, and a base 16. A mattress 40 is supported by base 16. The system 200 further includes a correction factor determination unit 210, display 220, air temperature controller 230, air set 240, humidity controller 250, RH set 260, main heater 270, and humidity heater 290.

Sensor 44 includes a humidity sensor and temperature sensor. However, separate humidity and temperature sensors may be used. The sensor 44 measures the air temperature and humidity in the enclosure. The corrected temperature value as calculated by the correction factor determination unit 210 and the humidity received from the sensor 44 are displayed on the display 220.

The correction factor determination unit 210 receives a measured temperature of the air and humidity in the enclosure from sensor 44. The correction factor determination unit 210 provides a corrected temperature value to air temperature controller 230, which adjusts the main heater 270 accordingly. The sensor 44 also provides the measured humidity to the humidity controller 250 which adjusts the humidity of the enclosure through the humidity heater 280.

The correction factor determination unit 210 provides a corrected temperature value based on the measured humidity and measured air temperature in the enclosure between hood 10 and base 16. The corrected temperature calculated by the correction factor determination unit 210 is the sum of the measured air temperature provided by sensor 44 and a correction factor, for example. In more detail, the correction factor is based on the measured humidity and is calculated with the following equation by the correction factor determination unit 210.

$$\text{Correction factor} = \alpha * RH[\%]^2 + \beta * RH[\%] + \gamma$$

where RH is the measured relative humidity. Further, $\alpha$ may be -0.000189, for example; $\beta$ may be 0.032867, for example; and $\gamma$ may be -1.8378, for example. Although, values are provided for $\alpha$, $\beta$, $\gamma$, these variables are not limited to these values the provided values. Also, with respect to the Correction factor formula, polynomials of an order other than quadratic may also be used.

Once the correction factor is determined by the correction factor determination unit 210, the correction factor determination unit 210 provides a control signal to the air temperature controller 230, which then adjusts the temperature of the air in the enclosure of the warming therapy apparatus 100 witch main heater 270.

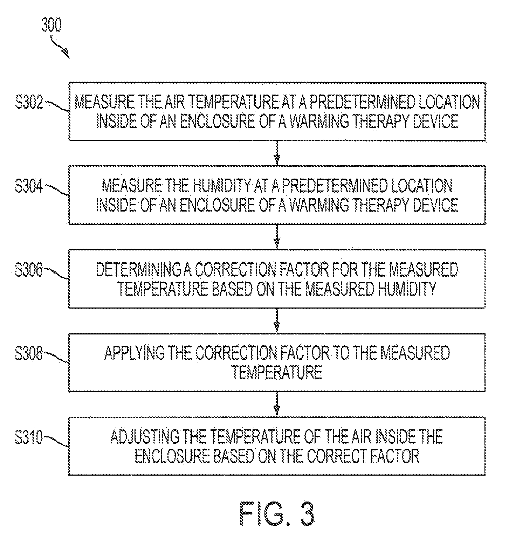

Referring to Figure 3, a process 300 for adjusting the temperature of the air within an enclosure of a warming therapy device is illustrated. The air temperature is measured in the enclosure between hood 10 and base 16 of the warming therapy apparatus 100 at a predetermined location (S302). The air temperature may be measured by sensor 44 as illustrated in Figure 2, for example. The humidity of the enclosure is also measured at a predetermined location inside the enclosure of the warming therapy apparatus 100 (S304). The humidity of the enclosure may also be measured by sensor 44, for example. A correction factor to apply to the measured air temperature is determined based on the measured humidity (S306). The correction factor determination unit 210 may determine the correction factor, for example. The correction factor is based on the measured relative humidity (RH) and is determined through use of the following equation:

$$\text{Correction factor} = \alpha * RH[\%]^2 + \beta * RH[\%] + \gamma$$

where RH is the measured relative humidity. Further, $\alpha$ may be -0.000189, for example; $\beta$ may be 0.032867, for example; and $\gamma$ may be -1.8378, for example. Although, values are provided for $\alpha$, $\beta$, $\gamma$, these variables are not limited to these values the provided values. Also, with respect to the Correction factor formula, polynomials of an order other than quadratic may also be used.

The determined correction factor is applied to the measured temperature to determine the corrected temperature (S308). For example, the correction factor is added to the measured temperature to arrive at the corrected temperature. This calculation is represented in the equation below.

$$\text{Corrected temperature} = \text{Correction Factor} + \text{Measured Air Temperature}$$

The temperature of the air inside the enclosure is adjusted to the corrected temperature. (S310). For example, the correction factor determination 210 may provide a control signal with the corrected temperature or temperature adjustment to the air temperature controller 230 which controls the main heater 270 to adjust the temperature of the enclosure to the corrected temperature.

Table 1 below shows how the air temperature as measured from the center mattress location 50 differs from the temperature of the air as measured by sensor 44 when the relative humidity of the enclosure changes. The center mattress location 50 corresponds to a position that a patient would be placed on a mattress within the enclosure of the warming therapy device 100. Table 1 also summarizes the corrected temperature calculated with techniques disclosed in the present disclosure and the corrected temperature calculated with the previous method. As will be seen in Chart 1 below, the corrected temperature calculated with embodiments of the present disclosure more accurately provides the temperature of the air in the enclosure and mirrors the temperature of the air measured at the center mattress location 50 providing an accurate temperature environment for a patient located on the mattress of the warming therapy device.

Table 1

| Relative Humidity (RH) [%] | Temperature as measured by sensor | Corrected temperature calculated with the previous method | Measured temperature at center mattress location | Corrected temperature calculated with embodiments of present disclosure |
|---|---|---|---|---|
| 24 | 31.2 | 30.1 | 30 | 30.0 |
| 30 | 31.2 | 30.1 | 30.3 | 30.2 |
| 40 | 31.1 | 30 | 30.5 | 30.3 |

(continued)

| Relative Humidity (RH) [%] | Temperature as measured by sensor | Corrected temperature calculated with the previous method | Measured temperature at center mattress location | Corrected temperature calculated with embodiments of present disclosure |
|---|---|---|---|---|
| 50 | 31.1 | 30 | 30.6 | 30.4 |
| 60 | 31.1 | 30 | 30.7 | 30.6 |
| 70 | 31.1 | 30 | 30.8 | 30.6 |
| 80 | 31.1 | 30 | 30.8 | 30.7 |
| 90 | 31.1 | 30 | 30.9 | 30.7 |
| 95 | 31.1 | 30 | 31 | 30.7 |

Chart 1 bellow shows a comparison of the air temperature measured at the center mattress location 50, the corrected air temperature based on a correction that is not a function of the relative humidity, or the previous method, and the corrected air temperature based on embodiments of the present disclosure. As can be seen, the corrected air temperature based on the measured relative humidity more accurately tracks the air temperature measured at the center mattress location 50 than the corrected air temperature based on a correction that is not a function of the relative humidity. Based on the more accurate prediction of the center mattress temperature measurement provided by the corrected air temperature, the benefits of the present invention can be appreciated.

Chart 1

Although the apparatus and methods have been described in connection with specific forms thereof, it will be appreciated that a wide variety of equivalents may be substituted for the specified elements described herein without departing from the scope of this disclosure as described in the appended claims.

**Claims**

1. A warming therapy apparatus (100), comprising:

a base (16) for supporting a medical patient;
a hood (10) surrounding a portion of the base (16);
a heater (270, 280) for heating the air inside the hood (10);
a sensor (44) for measuring an air temperature and a humidity inside the hood (10);

**characterized in that** it further comprises
a correction factor determination unit (210) which calculates the correction factor based on the measured humidity within the hood (10); and
a controller (230, 250) for adjusting the air temperature based on the correction factor.

2. The warming therapy apparatus (100) of claim 1, wherein the controller (230) adjusts the air temperature by changing the air temperature to the sum of the correction factor and the measured air temperature.

3. The warming therapy apparatus (100) of claim 1, wherein the correction factor determination unit (210) determines a corrected air temperature; and wherein the apparatus comprises an air temperature controller (230) which receives a control signal from the correction factor determination unit (210), and adjusts the air temperature accordingly.

4. The apparatus (100) of claim 3, wherein the correction factor determination unit (210) calculates the corrected air temperature by determining the sum of the measured air temperature and the calculated correction factor.

5. The apparatus (100) of any preceding claim, wherein the correction factor is a polynomial of the measured humidity.

6. The apparatus (100) of any preceding claim, wherein the correction factor is a quadratic polynomial of the measured humidity.

7. A method of adjusting the air temperature inside an enclosure (10) for medical patients, comprising:

measuring the air temperature at a predetermined location inside the enclosure (10);
measuring the humidity inside the enclosure (10);
determining a correction factor based on the measured humidity;
applying the correction factor to the measured air temperature to determine a temperature correction value; and
adjusting the air temperature inside the enclosure (10) based on the temperature correction value.

8. The method of claim 7, wherein the air temperature inside the enclosure is measured with a sensor (44).

9. The method of claim 7, wherein the humidity inside of the enclosure is measured with a sensor (44).

10. The method of claim 7, wherein the temperature correction value is the sum of the correction factor and the measured air temperature.

11. The method of claim 7, wherein the correction factor is a polynomial of the measured humidity.

12. The method of claim 7 wherein the correction factor is a quadratic polynomial of the measured humidity.

**Patentansprüche**

1. Wärmetherapievorrichtung (100), umfassend:

ein Unterteil (16) zum Tragen eines medizinischen Patienten;
eine Haube (10), die einen Abschnitt des Unterteils (16) umschließt;
eine Heizvorrichtung (270, 280) zur Erwärmung der Luft im Inneren der Haube (10);
einen Sensor (44) zum Messen einer Lufttemperatur und einer Luftfeuchtigkeit im Inneren der Haube (10);

**dadurch gekennzeichnet, dass** sie ferner eine Korrekturfaktor-Bestimmungseinheit (210) aufweist, welche den Korrekturfaktor basierend auf der gemessenen Luftfeuchtigkeit im Inneren der Haube (10) berechnet; und
eine Steuereinrichtung (230, 250) zum Einstellen der Lufttemperatur basierend auf dem Korrekturfaktor.

**2.** Wärmetherapievorrichtung (100) nach Anspruch 1, wobei die Steuereinrichtung (230) die Lufttemperatur durch Veränderung der Lufttemperatur auf die Summe aus dem Korrekturfaktor und der gemessenen Lufttemperatur einstellt.

**3.** Wärmetherapievorrichtung (100) nach Anspruch 1, wobei die Korrekturfaktor-Bestimmungseinheit (210) eine korrigierte Lufttemperatur bestimmt; und wobei die Vorrichtung einen Lufttemperaturregler (230) aufweist, der ein Steuersignal von der Korrekturfaktor-Bestimmungseinheit (210) empfängt, und die Lufttemperatur entsprechend einstellt.

**4.** Vorrichtung (100) nach Anspruch 3, wobei die Korrekturfaktor-Bestimmungseinheit (210) die korrigierte Lufttemperatur durch Bestimmung der Summe aus der gemessenen Lufttemperatur und dem berechneten Korrekturfaktor berechnet.

**5.** Vorrichtung (100) nach einem der vorhergehenden Ansprüche, wobei der Korrekturfaktor ein Polynom der gemessenen Luftfeuchtigkeit ist.

**6.** Vorrichtung (100) nach einem der vorhergehenden Ansprüche, wobei der Korrekturfaktor ein quadratisches Polynom der gemessenen Luftfeuchtigkeit ist.

**7.** Verfahren zum Einstellen der Lufttemperatur im Inneren eines Gehäuses (10) für medizinische Patienten, umfassend:

Messen der Lufttemperatur an einer vorbestimmten Stelle im Inneren des Gehäuses (10);
Messen der Luftfeuchtigkeit im Inneren des Gehäuses (10);
Bestimmen eines Korrekturfaktors basierend auf der gemessenen Luftfeuchtigkeit;
Anwenden des Korrekturfaktors auf die gemessene Lufttemperatur, um einen Temperaturkorrekturwert zu bestimmen; und
Einstellen der Lufttemperatur innerhalb des Gehäuses (10) basierend auf dem Temperaturkorrekturwert.

**8.** Verfahren nach Anspruch 7, wobei die Lufttemperatur im Inneren des Gehäuses mit einem Sensor (44) gemessen wird.

**9.** Verfahren nach Anspruch 7, wobei die Luftfeuchtigkeit im Inneren des Gehäuses mit einem Sensor (44) gemessen wird.

**10.** Verfahren nach Anspruch 7, wobei der Temperaturkorrekturwert die Summe aus dem Korrekturfaktor und der gemessenen Lufttemperatur ist.

**11.** Verfahren nach Anspruch 7, wobei der Korrekturfaktor ein Polynom der gemessenen Luftfeuchtigkeit ist.

**12.** Verfahren nach Anspruch 7, wobei der Korrekturfaktor ein quadratisches Polynom der gemessenen Luftfeuchtigkeit ist.

**Revendications**

**1.** Appareil de thérapie de réchauffement (100), comprenant :

une base (16) destinée à supporter un patient médical,
un capot (10) entourant une partie de la base (16),
un dispositif de chauffage (270, 280) destiné à réchauffer l'air à l'intérieur du capot (10),
un capteur (44) destiné à mesurer une température d'air et une humidité à l'intérieur du capot (10),

**caractérisé en ce qu'**il comprend en outre
une unité de détermination de facteur de correction (210) qui calcule le facteur de correction sur la base de l'humidité mesurée à l'intérieur du capot (10), et
un contrôleur (230, 250) destiné à ajuster la température d'air sur la base du facteur de correction.

**2.** Appareil de thérapie de réchauffement (100) selon la revendication 1, dans lequel le contrôleur (230) ajuste la température d'air en modifiant la température d'air à la somme du facteur de correction et de la température d'air mesurée.

**3.** Appareil de thérapie de réchauffement (100) selon la revendication 1, dans lequel l'unité de détermination de facteur de correction (210) détermine une température d'air corrigée, et où l'appareil comprend un contrôleur de température d'air (230) qui reçoit un signal de commande provenant de l'unité de détermination de facteur de correction (210), et ajuste la température d'air en conséquence.

**4.** Appareil (100) selon la revendication 3, dans lequel l'unité de détermination de facteur de correction (210) calcule la température d'air corrigée en déterminant la somme de la température d'air mesurée et du facteur de correction calculé.

**5.** Appareil (100) selon l'une quelconque des revendications précédentes, dans lequel le facteur de correction est un polynôme de l'humidité mesurée.

**6.** Appareil (100) selon l'une quelconque des revendications précédentes, dans lequel le facteur de correction est un polynôme du second degré de l'humidité mesurée.

**7.** Procédé d'ajustement de la température d'air à l'intérieur d'une enceinte (10) pour des patients médicaux, comprenant les étapes consistant à :

mesurer la température d'air à une position prédéterminée à l'intérieur de l'enceinte (10),
mesurer l'humidité à l'intérieur de l'enceinte (10),
déterminer un facteur de correction sur la base de l'humidité mesurée,
appliquer le facteur de correction à la température d'air mesurée pour déterminer une valeur de correction de température, et
ajuster la température d'air à l'intérieur de l'enceinte (10) sur la base de la valeur de correction de température.

**8.** Procédé selon la revendication 7, dans lequel la température d'air à l'intérieur de l'enceinte est mesurée avec un capteur (44).

**9.** Procédé selon la revendication 7, dans lequel l'humidité à l'intérieur de l'enceinte est mesurée avec un capteur (44).

**10.** Procédé selon la revendication 7, dans lequel la valeur de correction de température est la somme du facteur de correction et de la température d'air mesurée.

**11.** Procédé selon la revendication 7, dans lequel le facteur de correction est un polynôme de l'humidité mesurée.

**12.** Procédé selon la revendication 7 dans lequel le facteur de correction est un polynôme du second degré de l'humidité mesurée.

FIG. 1

FIG. 2

EP 2 775 983 B1

*300*

| S302 | MEASURE THE AIR TEMPERATURE AT A PREDETERMINED LOCATION INSIDE OF AN ENCLOSURE OF A WARMING THERAPY DEVICE |
| S304 | MEASURE THE HUMIDITY AT A PREDETERMINED LOCATION INSIDE OF AN ENCLOSURE OF A WARMING THERAPY DEVICE |
| S306 | DETERMINING A CORRECTION FACTOR FOR THE MEASURED TEMPERATURE BASED ON THE MEASURED HUMIDITY |
| S308 | APPLYING THE CORRECTION FACTOR TO THE MEASURED TEMPERATURE |
| S310 | ADJUSTING THE TEMPERATURE OF THE AIR INSIDE THE ENCLOSURE BASED ON THE CORRECT FACTOR |

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5730355 A